# EUROPÄISCHE PATENTSCHRIFT

(11) **EP 1 292 264 B1**
(45) Veröffentlichungstag und Bekanntmachung des Hinweises auf die Patenterteilung: **03.01.2007**
(21) Anmeldenummer: 01943490.1
(22) Anmeldetag: 09.06.2001
(51) Int. Cl.: A61K 8/41, A61K 8/67, A61Q 5/06

(54) **MITTEL ZUM FÄRBEN BZW. TÖNEN UND GLEICHZEITIGEM PFLEGEN VON HAAREN**
AGENT FOR DYEING OR COLORING AND SIMULTANEOUSLY PROTECTING HAIR
AGENT DE COLORATION CAPILLAIRE POUVANT TEINDRE ET SOIGNER LES CHEVEUX SIMULTANEMENT

(30) Priorität: 20.06.2000 DE 10030313; 26.04.2001 DE 10120304
(43) Veröffentlichungstag der Anmeldung: 19.03.2003
(73) Patentinhaber: Henkel Kommanditgesellschaft auf Aktien, 40589 Düsseldorf (DE)
(72) Erfinder: KLEEN, Astrid, 40699 Erkrath (DE); NAUMANN, Frank, 40219 Düsseldorf (DE); HÖFFKES, Horst, 40595 Düsseldorf (DE)
(86) Internationale Anmeldenummer: PCT/EP2001/006560
(87) Internationale Veröffentlichungsnummer: WO 2001/097757

(56) Entgegenhaltungen:
- EP-A- 0 873 744
- EP-A- 1 101 823
- DE-A- 19 955 915
- JP-A- 6 183 934
- CHEMICAL ABSTRACTS, vol. 107, no. 20, 16. November 1987 (1987-11-16) Columbus, Ohio, US; abstract no. 183330, H.KOJIMA, J.TAKENAKA: "Hair preparations containing reducing agents, sequestering agents, metallic salts, dyes and oxidizing agents" XP002183446 & JP 62 132813 A (NISHIRENJI TRADING) 16. Juni 1987 (1987-06-16)
- CHEMICAL ABSTRACTS, vol. 107, no. 20, 16. November 1987 (1987-11-16) Columbus, Ohio, US; abstract no. 183331, H.KOJIMA, J.TAKENAKA: "Hair preparations containing reducing agents, metallic salts, dyes and oxidizing agents" XP002184002 & JP 62 132814 A (SANSHIDO DEIYAKU) 16. Juni 1987 (1987-06-16)
- CHEMICAL ABSTRACTS, vol. 110, no. 14, 3. April 1989 (1989-04-03) Columbus, Ohio, US; abstract no. 121002, H.KOJIMA, J.TAKENAKA: "Hair preparations for wave setting and dyeing at the same time" XP002184003 & JP 63 014712 A (SANSHIDO SEIYAKU) 21. Januar 1988 (1988-01-21)
- CHEMICAL ABSTRACTS, vol. 112, no. 8, 19. Februar 1990 (1990-02-19) Columbus, Ohio, US; abstract no. 62357, H.KOJIMA, J.TAKENAKA: "Simultaneous procedure for hair dyeing and wave setting" XP002184004 & JP 01 066109 A (SANSHIDO SEIYAKU) 13. März 1989 (1989-03-13)
- PATENT ABSTRACTS OF JAPAN vol. 0185, no. 28, 6. Oktober 1994 (1994-10-06) & JP 06 183934 A (HOYU), 5. Juli 1994 (1994-07-05)

## Beschreibung

Die Erfindung betrifft ein Mittel und ein Verfahren zum Färben bzw. Tönen und Pflegen von Keratinfasern, insbesondere dem menschlichen Haar, sowie die Verwendung von Vitamin B6-Derivaten in einem entsprechenden Verfahren.

Zubereitungen zum Tönen und Färben von Haaren sind ein wichtiger Typ von kosmetischen Mitteln. Sie können dazu dienen, die natürliche Haarfarbe gemäß den Wünschen der entsprechenden Person leicht oder stärker zu nuancieren, eine gänzlich andere Haarfarbe zu erzielen oder unerwünschte Farbtöne, wie beispielsweise Grautöne, zu überdecken. Übliche Haarfärbemittel werden, je nach gewünschter Farbe bzw. Dauerhaftigkeit der Färbung, entweder auf Basis von Oxidationsfarbstoffen oder auf Basis von direktziehenden Farbstoffen formuliert. Häufig werden auch Kombinationen von Oxidationsfarbstoffen und direktziehenden Farbstoffen zur Erzielung spezieller Nuancen eingesetzt.

Gute Farbstoffe zeichnen sich durch hohe Farbstärke aus. Weiterhin sind gute Schweiß-, Wärme-, Dauerwell-, Wasch- und Lichtechtheit gewünscht. Ferner sollten sie in toxikologischer und dermatologischer Hinsicht unbedenklich sein. Es ist auch von Vorteil, wenn die Substanzen eine hohe Löslichkeit in verschiedenen Basisformulierungen besitzen.

Färbemittel auf Basis von Oxidationsfarbstoffen führen zu brillanten und dauerhaften Farbtönen. Sie bedingen allerdings den Einsatz starker Oxidationsmittel wie beispielsweise Wasserstoffperoxid-Lösungen. Dies kann das zu färbende Haar schädigen. Kontakte der Haut mit diesen Färbemitteln können bei sehr empfindlichen Personen zu unerwünschten Reaktionen führen.

Färbemittel auf der Basis direktziehender Farbstoffe kommen ohne Oxidationsmittel aus und können auch bei pH-Werten im Bereich des Neutralpunktes formuliert werden.

Neben dem Färben bzw. Tönen des Haars ist die Haarpflege ein wichtiger Aspekt der Haarkosmetik. So kann die Anwendung von haarkosmetischen Formulierungen mit keratinstrukturierenden Wirkstoffen den schädigenden Einflüssen, welche z.B. durch alkalische und/oder keratinreduzierende Präparate hervorgerufen werden, entgegengewirken.
Pyridoxin und weitere Verbindungen der Vitamin B6-Gruppe sind als Komponenten in Haartonika zur Verringerung des Nachfettens und zur Stimulierung des Haarwuchses erwähnt worden. In EP 0678293 A2 werden topische Zusammensetzungen mit einem Gehalt von Pyridoxintripropionat zur Behandlung des Haars und der Haut vorgeschlagen. In EP 001079 A1 sind kosmetische Zusammensetzungen mit antiseborrhoischer Wirkung beschrieben, die Pyridoxin-tripalmitat als Wirkstoff enthalten
Haarfärbemittel, die Derivate des Pyridoxins, Pyridoxals oder Pyridoxamins als wirksamen, die Keratinstruktur verbessernden Zusatzstoff enthalten, sind dem Fachmann bisher nicht bekannt.

Es wurde nunmehr überraschenderweise gefunden, daß der Einsatz von Derivaten des Pyridoxins, Pyridoxals und Pyridoxamins in einem Färbemittel mit mindestens einem direktziehenden Farbstoff eine restrukturierende Wirkung auf das Haarkeratin ausübt. Diese Wirkung entfaltet sich auch während des Färbevorgangs.

Der erste Gegenstand der Erfindung ist daher ein Mittel zum Färben von Keratinfasern, insbesondere dem menschlichen Haar, enthaltend mindestens einen direktziehenden Farbstoff, dadurch gekennzeichnet, daß das Färbemittel zusätzlich mindestens eine Verbindung der Formel (I) 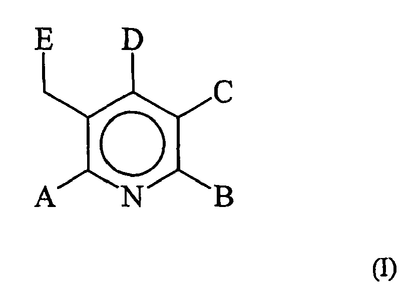 worin
- A und B stehen unabhängig voneinander für Wasserstoff, Halogen, eine C₁-C₄-Alkylgruppe, eine C₃-C₆-Cycloalkylgruppe, eine C₁-C₄-Monohydroxyalkylgruppe, eine C₂-C₄-Oligohydroxyalkylgruppe, eine C₁-C₄-Aminoalkylgruppe, eine Gruppe -OR oder eine Gruppe -NR¹R², in der R¹ und R² unabhängig voneinander für Wasserstoff, eine C₁-C₄-Alkylgruppe oder eine C₁-C₄-Monohydroxyalkylgruppe stehen, oder R¹ und R² zusammen mit dem Stickstoffatom einen gesättigten Ring bilden,
- C steht für eine Gruppe -OR, -NR¹R², -OP(O)(OR³)₂, eine C₁-C₄-Monohydroxyalkylgruppe, eine C₂-C₄-Oligohydroxyalkylgruppe oder eine C₁-C₄-Alkylgruppe,
- D steht für eine Gruppe -OR, eine Carboxygruppe, eine C₁-C₂₂-Alkoxycarbonylgruppe, einen Formylrest, eine Gruppe -CH₂OR oder eine Gruppe - CH₂-NR₂,
- E steht für eine Gruppe -OR, -OP(O)(OR³)₂, eine C₁-C₄-Monohydroxyalkylgruppe oder eine C₂-C₄-Oligohydroxyalkylgruppe,
wobei
- R jeweils steht für Wasserstoff, eine C₁-C₄-Alkylgruppe, eine C₁-C₂₂-Acylgruppe, eine Hydroxy-C₂-C₂₂-acylgruppe, eine C₂-C₁₀-Carboxyacylgruppe, eine C₃-C₁₀-Oligocarboxyacylgruppe, eine Oligocarboxymonohydroxy-C₃-C₁₀-acylgruppe, eine Oligocarboxy-oligohydroxy-C₃-C₁₀-acylgruppe, eine C₃-C₈-Cycloalkylgruppe, eine C₁-C₄-Monohydroxyalkylgruppe, eine C₂-C₄-Oligohydroxyalkylgruppe, eine Arylgruppe, welche eine Hydroxy-, Nitro- oder Aminogruppe enthalten kann, einen heteroaromatischen Rest oder eine Gruppe -CH₂CH₂NR¹R², in der R¹ und R² wie oben definiert sind,
- R³ jeweils steht für Wasserstoff oder eine C₁-C₅-Alkylgruppe,
oder eines der entsprechenden physiologisch verträglichen Salze enthält.

Verbindungen der Formel (I) werden bevorzugt, in denen eine der beiden Gruppen A oder B für Wasserstoff steht. Besonders bevorzugte Verbindungen der Formel (I) sind solche, in denen eine der beiden Gruppen A oder B Wasserstoff und die andere Gruppe eine C₁-C₄-Alkylgruppe ist.

Weiterhin sind Verbindungen der Formel (I) bevorzugt, in denen C für eine Hydroxygruppe, eine C₁-C₄-Monohydroxyalkylgruppe oder eine C₂-C₄-Oligohydroxyalkylgruppe steht.

Verbindungen der Formel (I) werden bevorzugt, in denen D für eine Hydroxymethylgruppe, eine Hydroxygruppe, eine Carboxygruppe, eine Gruppe -CH₂-NR₂ oder einen Formylrest steht.

Ebenfalls bevorzugte Verbindungen der Formel (I) sind solche, in denen E für eine Hydroxygruppe, eine C₁-C₄-Monohydroxyalkylgruppe oder eine Gruppe -OP(O)(OH)₂ steht.

Besonders bevorzugte Verbindungen nach Formel I sind Pyridoxin (A = H, B = CH₃, C = OH, D = CH₂OH, E = OH), Pyridoxal (A = H, B = CH₃, C = OH, D = CHO, E = OH), Pyridoxal-5'-phosphat (A = H, B = CH₃, C = OH, D = CHO, E = OP(O)(OH)₂) und Pyridoxamin (A = H, B = CH₃, C = OH, D = CH₂NH₂, E = OH).

Beispiele für C₁-C₄-Alkylgruppen in den erfindungsgemäßen Verbindungen sind Methyl, Ethyl, n-Propyl, Isopropyl, n-Butyl und tert-Butyl. Bevorzugte Alkylgruppen sind Methyl und Ethyl, Methyl ist eine besonders bevorzugte Alkylgruppe. Bevorzugte C₃-C₆-Cycloalkylgruppen sind Cyclopropyl, Cyclobutyl, Cyclopentyl, Cyclohexyl. Cyclohexyl und Cyclopentyl sind besonders bevorzugte Gruppen. Bevorzugte C₁-C₄-Monohydroxyalkylgruppen sind die Gruppen Hydroxymethyl, 2-Hydroxyethyl, 3-Hydroxypropyl oder 4-Hydroxybutyl; Hydroxymethyl und 2-Hydroxyethyl sind besonders bevorzugte Monohydroxyalkylgruppen. Eine bevorzugte C₂-C₄-Oligohydroxyalkylgruppe ist die 1,2-Dihydroxyethylgruppe. Bevorzugte C₁-C₂₂-Acylgruppen sind beispielsweise Acetyl, Propionyl, Butyryl, Valeryl, Capryl, Lauryl, Myristyl, Palmityl, Stearyl, Linolyl, Behenyl. Beispiele für eine Hydroxy-C₂-C₂₂-acylgruppe sind Salicylsäure oder Milchsäure. Bevorzugte C₂-C₁₀-Carboxyacylgruppen leiten sich beispielsweise ab von der Malonsäure, Bernsteinsäure oder Adipinsäure. Ein Beispiel für eine bevorzugte C₃-C₁₀-Oligocarboxyacylgruppe ist die Glycerinsäure. Eine bevorzugte eine Oligocarboxy-monohydroxy-C₃-C₁₀-acylgruppe leitet sich z.B. von der Zitronensäure oder Äpfelsäure ab. Bevorzugte Oligocarboxy-oligohydroxy-C₃-C₁₀-acylgruppen sind z.B. abgeleitet aus Weinsäure. Als Halogensubstituenten eignen sich erfindungsgemäß bevorzugt Fluor, Chlor, Brom und Iod, besonders bevorzugt sind Chlor und Brom. Unter physiologisch verträglichen Salzen werden Salze anorganischer oder organischer Säuren, z. B. Hydrochloride, Sulfate oder Hydrobromide, verstanden. Die weiteren verwendeten Begriffe leiten sich erfindungsgemäß von den hier gegebenen Definitionen ab.

Die Ester-Derivate der Verbindungen gemäß Formel (I) weisen auch physiologische und die Haarstruktur verbessernde Eigenschaften auf. Dies gilt insbesondere für die Ester des Pyridoxins, die durch Hydrolyse in das Pyridoxin übergehen können. Darüber hinaus erlangen die Esterderivate eine verbesserte Lipidlöslichkeit verglichen mit den nicht veresterten Derivaten. Weitere Beispiele für Carbonsäure-Esterderivate des Pyridoxins leiten sich ab von den Carbonsäuren wie Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure, sowie von den Dicarbonsäuren ausgewählt aus der Gruppe, die gebildet wird durch Verbindungen der allgemeinen Formel (II), 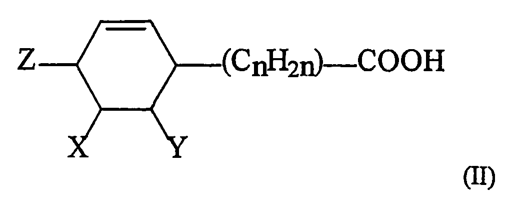 in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (II), die zusätzlich noch 1 bis 3 Methyl- oder Ethylsubstituenten am Cyclohexenring tragen sowie Dicarbonsäuren, die aus den Dicarbonsäuren gemäß Formel (II) formal durch Anlagerung eines Moleküls Wasser an die Doppelbindung im Cyclohexenring entstehen.

Die erfindungsgemäßen Mittel enthalten die Verbindung der Formel (I) bevorzugt in Mengen von 0,05 bis 5,0 Gew.-%, bezogen auf das Färbemittel. Mengen von 0,1 bis 2 Gew.-% sind besonders bevorzugt.

Gemäß einer ersten bevorzugten Ausführungsform der Erfindung handelt es sich um solche Mittel, die nur eine vorübergehende Färbung der Faser bewirken sollen. Solche Mittel werden häufig als Tönungsmittel bezeichnet. Diese Ausführungsform umfaßt beispielsweise auch solche Haarbehandlungsmittel, mit denen die Haare nicht nur vorübergehend gefärbt, sondern auch zu einer bestimmten Frisur geformt werden sollen. In diesem Falle spricht man von Tönungsfestigern.

Da die Ausfärbung der keratinischen Fasern mit Tönungsmitteln üblicherweise ohne die Zuhilfenahme von oxidierenden Komponenten, insbesondere Wasserstoffperoxid, erfolgt, sind die erfindungsgemäßen Mittel gemäß dieser Ausführungsform bevorzugt frei von Oxidationsfarbstoffvorprodukten.

Die erfindungsgemäßen Mittel können als Färbecreme, Tinktur, Lotion, Schaum oder als Aerosol vorliegen.

Die Erfindung umfaßt Mittel, die neben der Verbindung gemäß Formel (I) noch einen direktziehenden Farbstoff enthalten. Gemäß einer bevorzugten Ausführungsform enthalten die erfindungsgemäßen Mittel mehrere, insbesondere 2 - 10 direktziehende Farbstoffe.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 12, Disperse Blue 3, HC Violet 1, Disperse Violet 1, Disperse Violet 4, Acid Violet 43, Disperse Black 9 und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ebenfalls als direktziehende Farbstoffe erfindungsgemäß einsetzbar sind kationische direktziehende Farbstoffe. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatische Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908 in den Ansprüchen 6 bis 11 offenbart werden und wie sie in den Druckschriften WO 95/01772 und WO 95/15144 beschrieben werden. Auf die Offenbarung in den genannten Druckschriften wird ausdrücklich Bezug genommen.

Bevorzugte kationische direktziehende Farbstoffe der Gruppe (c) sind insbesondere die folgenden Verbindungen:

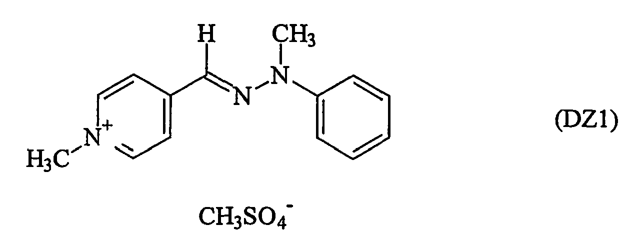

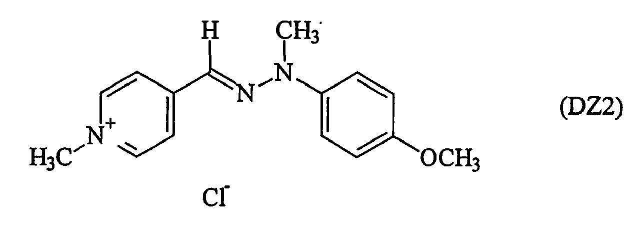

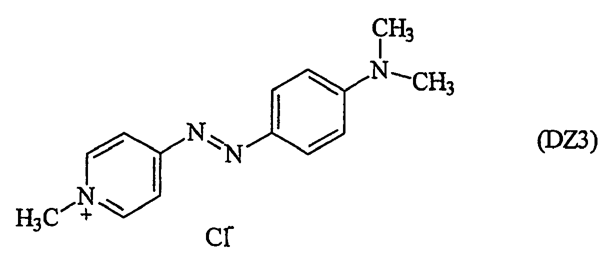

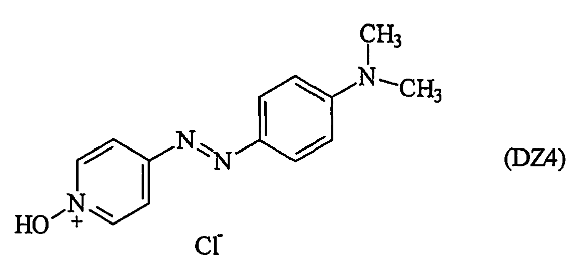

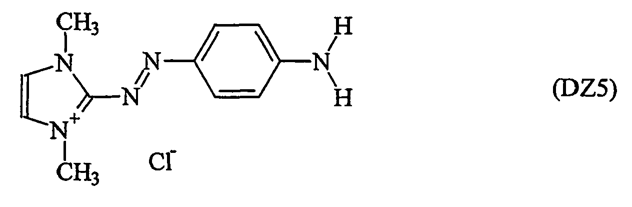

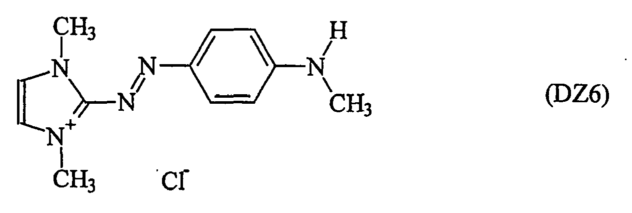

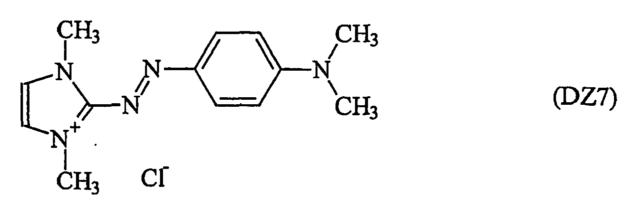

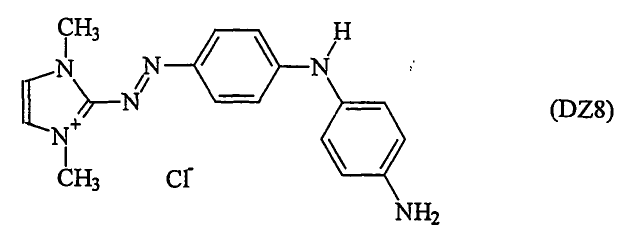

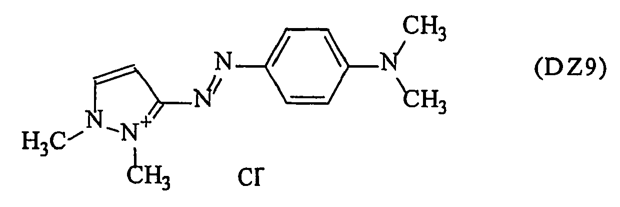

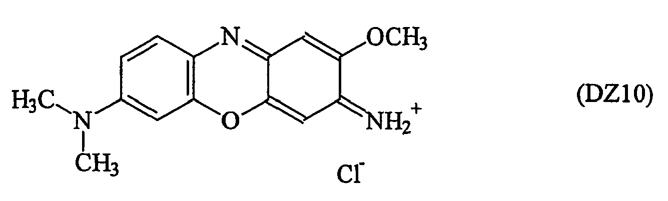

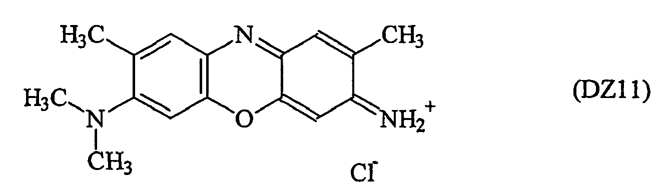

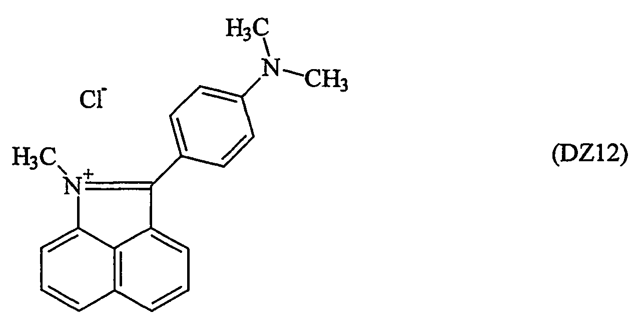

Die Verbindungen der Formeln (DZ1), (DZ5) und (DZ7) sind ganz besonders bevorzugte kationische direktziehende Farbstoffe der Gruppe (c). Die kationischen direktziehenden Farbstoffe, die unter dem Warenzeichen Arianor^{®} vertrieben werden, sind erfindungsgemäß besonders bevorzugte direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur auftretende Farbstoffe enthalten, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind.

Gemäß einer zweiten bevorzugten Ausführungsform enthalten die erfindungsgemäßen Mittel zusätzlich mindestens ein Oxidationsfarbstoff-Vorprodukt vom Typ der Entwickler. Diese, in der Regel farblosen, Verbindungen reagieren unter der Einwirkung von Oxidationsmitteln oder von Luftsauerstoff, gegebenenfalls mit Hilfe spezieller Enzyme oder Metallionen als Katalysator, mit sich unter der Ausbildung der gewünschten Farbstoffe. Insbesondere zur Ausbildung natürlicher Haarfarbtöne werden aber in der Regel Kombinationen von mehreren Entwicklerkomponenten eingesetzt. Weiterhin werden in der Regel zusätzlich sogenannte Kuppler eingesetzt, die unter dem Einfluß von Oxidationsmitteln mit den Entwicklerkomponenten reagieren, was zu neuen Farben bzw. einer Nuancierung der Farbe führt. Erfindungsgemäß können sowohl eine als auch mehrere Kupplerkomponenten in Kombination mit einer oder mehreren Entwicklerkomponenten eingesetzt werden.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, N,N'-Bis-(4'-aminophenyl)-1,4-diazacycloheptan, 1,3-Bis(N(2-hydroxyethyl)-N(4-aminophenyl-amino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 931 bzw. WO 94/08970 wie z.B. 4,5-Diamino-1-(2'-hydroxyethyl)-pyrazol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Die erfindungsgemäßen Mittel dieser Ausführungsform enthalten außer den Verbindungen gemäß Formel (I), den Entwickler- und gegebenenfalls Kupplerkomponenten bevorzugt mehrere direktziehende Farbstoffe. Es sei an dieser Stelle auf das oben gesagte verwiesen.

Weiterhin können in den erfindungsgemäßen Färbemitteln Vorläufer natürlicher Farbstoffe, insbesondere Indole und Indoline, sowie deren physiologisch verträgliche Salze, enthalten sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol-6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

Es ist nicht erforderlich, daß die Oxidationsfarbstoffvorprodukte, die direktziehenden Farbstoffe oder die Vorläufer natürlicher Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und Tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Es ist möglich, die Oxidation mit Hilfe von Enzymen durchzuführen. Dabei können die Enzyme (Enzymklasse 1: Oxidoreduktasen) Elektronen aus geeigneten Entwicklerkomponenten (Reduktionsmittel) auf Luftsauerstoff übertragen. Bevorzugt wären Oxidasen wie Tyrosinase und Laccase aber auch Glucoseoxidase, Uricase oder Pyruvatoxidase. Weiterhin können die Enzyme zur Verstärkung der Wirkung geringer Mengen vorhandener Oxidationsmittel dienen. Ein Beispiel für ein derartiges enzymatisches Verfahren stellt das Vorgehen dar, die Wirkung geringer Mengen (z.B. 1% und weniger, bezogen auf das gesamte Mittel) Wasserstoffperoxid durch Peroxidasen zu verstärken.

Die erfindungsgemäßen Mittel können weiterhin alle für solche Zubereitungen bekannten Wirk-, Zusatz- und Hilfsstoffe enthalten. In vielen Fällen enthalten diese Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R'O-(Z)ₓ. Diese Verbindungen sind durch die folgenden Parameter gekennzeichnet.

Der Alkylrest R' enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R'
- im wesentlichen aus C₈- und C₁₀-Alkylgruppen,
- im wesentlichen aus C₁₂- und C₁₄-Alkylgruppen,
- im wesentlichen aus C₈-C₁₆-Alkylgruppen oder
- im wesentlichen aus C₁₂-C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quatemium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethyl-ammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können die erfindungsgemäßen Mittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat^{®}100 (Poly(dimethyldiallylammoniumchlorid)), Merquat^{®}550 (Dimethyldiallylammoniumchlorid-Acrylamid-Copolymer) und Merquat^{®} 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich.
- Vinylpyrrolidon-Methoimidazoliniumchlorid-Copolymere, wie sie unter der Bezeichnung Luviquat^{®} angeboten werden.
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Alternativ zu den kationischen Polymeren werden als konditionierende Wirkstoffe zwitterionische oder ampholytische Polymere besonders bevorzugt eingesetzt. Bevorzugte Vertreter sind Octylacrylamid/Methylmethacrylat/tert-Butylaminoethylmethacrylat/2-Hydroxypropylmethacrylat Copolymere und insbesondere das Acrylamidopropyl-trimethylammoniumchlorid/Acrylat Copolymer.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien.
   Bezüglich weiterer fakultativer Komponenten sowie den eingesetzten Mengen dieser Komponenten wird ausdrücklich auf die dem Fachmann bekannten einschlägigen Handbücher, z. B. K. Schrader, Grundlagen und Rezepturen der Kosmetika, 2. Auflage, Hüthig Buch Verlag, Heidelberg, 1989, verwiesen.
   Ein zweiter Gegenstand der Erfindung ist ein Verfahren zum Tönen und/oder Färben von Keratinfasern, insbesondere menschlichen Haaren, in dem
- gewünschtenfalls ein Vorbehandlungsmittel M1 auf die Faser aufgebracht wird, dann
- ein Tönungs- oder Färbemittel M2 auf der Faser zur Anwendung kommt, wobei gewünschtenfalls dem Mittel M2 vor der Anwendung ein weiteres Mittel M3 zugegeben wird
- dieses Färbemittel M2 nach einer Zeit von 5-30 Minuten von der Faser abgespült wird,
- und nach der Behandlung gegebenenfalls ein Nachbehandlungsmittel M4 auf die Faser aufgetragen und nach einer Einwirkzeit von einigen Minuten wieder abgespült wird,
wobei mindestens eines der Mittel M1, M2, M3 oder M4 mindestens eine Verbindung gemäß Formel (I) enthält.

Das in diesem Verfahren verwendete Mittel M1 ist ein Vorbehandlungsmittel, enthaltend mindestens eine Verbindung gemäß Formel (I) in einem kosmetischen Träger. Das Färbemittel M2 ist ein Mittel gemäß einem der Ansprüche 1 bis 9. Es ist bevorzugt, dem Mittel M2 kurz vor der Anwendung ein Mittel M3, enthaltend mindestens eine Verbindung gemäß Formel (I), zuzufügen. Das Mittel M3, enthaltend mindestens eine Verbindung der Formel (I), kann sowohl als Feststoff als auch als wäßrige Lösung konfektioniert sein.
Das Nachbehandlungsmittel M4 ist erfindungsgemäß ein Haarnachbehandlungsmittel, wie es z.B. in K. Schrader, Grundlagen und Rezepturen der Kosmetika, 2. Auflage, 1989, Dr. Alfred Hüthig Verlag, Heidelberg S. 722 ff. beschrieben wird, welches gegebenenfalls mindestens eine Verbindung gemäß Formel (I) enthält.

Ein dritter Gegenstand der Erfindung ist die Verwendung eines Mittels in einem Verfahren zum Färben bzw. Tönen von Keratinfasern, insbesondere dem menschlichen Haar, das mindestens einen direktziehenden Farbstoff und zusätzlich mindestens eine Verbindung gemäß Formel (I) enthält, 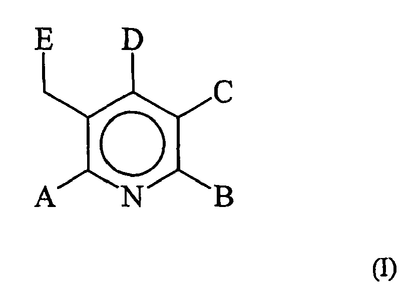 worin
- A und B stehen unabhängig voneinander für Wasserstoff, Halogen, eine C₁-C₄-Alkylgruppe, eine C₃-C₆-Cycloalkylgruppe, eine C₁-C₄-Monohydroxyalkylgruppe, eine C₂-C₄-Oligohydroxyalkylgruppe, eine C₁-C₄-Aminoalkylgruppe, eine Gruppe -OR oder eine Gruppe -NR¹R², in der R¹ und R² unabhängig voneinander für Wasserstoff, eine C₁-C₄-Alkylgruppe oder eine C₁-C₄-Monohydroxyalkylgruppe stehen, oder R¹ und R² zusammen mit dem Stickstoffatom einen gesättigten Ring bilden,
- C steht für eine Gruppe -OR, -NR¹R², -OP(O)(OR³)₂, eine C₁-C₄-Monohydroxyalkylgruppe, eine C₂-C₄-Oligohydroxyalkylgruppe oder eine C₁-C₄-Alkylgruppe,
- D steht für eine Gruppe -OR, eine Carboxygruppe, eine C₁-C₂₂-Alkoxycarbonylgruppe, einen Formylrest, eine Gruppe -CH₂OR oder eine Gruppe - CH₂-NR₂,
- E steht für eine Gruppe -OR, -OP(O)(OR³)₂, eine C₁-C₄-Monohydroxyalkylgruppe oder eine C₂-C₄-Oligohydroxyalkylgruppe,
wobei
- R jeweils steht für Wasserstoff, eine C₁-C₄-Alkylgruppe, eine C₁-C₂₂-Acylgruppe, eine Hydroxy-C₂-C₂₂-acylgruppe, eine C₂-C₁₀-Carboxyacylgruppe, eine C₃-C₁₀-Oligocarboxyacylgruppe, eine Oligocarboxymonohydroxy-C₃-C₁₀-acylgruppe, eine Oligocarboxy-oligohydroxy-C₃-C₁₀-acylgruppe, eine C₃-C₈-Cycloalkylgruppe, eine C₁-C₄-Monohydroxyalkylgruppe, eine C₂-C₄-Oligohydroxyalkylgruppe, eine Arylgruppe, welche eine Hydroxy-, Nitro- oder Aminogruppe enthalten kann, einen heteroaromatischen Rest oder eine Gruppe -CH₂CH₂NR¹R², in der R¹ und R² wie oben definiert sind,
- R³ jeweils steht für Wasserstoff oder eine C₁-C₅-Alkylgruppe,
oder eines der entsprechenden physiologisch verträglichen Salze enthält.

Die nachfolgenden Beispiele sollen den Erfindungsgegenstand näher erläutern.

### Beispiele

### I) Ausfärbungen

Es wurden Färbemittel mit zwei unterschiedlichen Trägern formuliert.

### FÄRBEMITTEL 1

### Teilmischung A

| | |
|---|---|
| Hydrenol^{®}D¹ | 3,00g |
| Kokoslorol^{®}C12-18² | 2,50g |
| Akypo^{®} RLM 45N³ | 3,00g |
| p-Hydroxybenzoesäurepropylester | 0,15g |
| p-Hydroxybenzoesäuremethylester | 0,15g |
| Phenoxyethanol | 0,50g |
| Carbopol^{®}ETD 2001⁴ | 0,20g |
| Vitamin E-Acetat | 0,20g |
| AMP 95⁵ | ad pH=7,0 |
| Wasser | 70,00g |

| | |
|---|---|
| ¹ C₁₆-C₁₈-Fettalkohol (INCI-Bezeichnung: Cetearyl alcohol) (COGNIS) ² C₁₂-C₁₈-Fettalkohol (INCI-Bezeichnung: Coconut alcohol) (COGNIS) ³ Laurylalkohol mit ca. 4,5mol Ethylenoxid-Essigsäure-Natriumsalz (ca. 82% Aktivsubstanz (INCI-Bezeichnung: Sodium Laureth-6 Carboxylate)) (KAO, Chem-Y) ⁴ Polyacrylsäure Homopolymer (INCI-Bezeichnung: Carbomer) (GOODRICH) ⁵ 2-Amino-2-methylpropanol (95% Aktivsubstanz, 5% Wasser) (CSC CHEMIE) | |

Die Substanzen wurden bei 80°C aufgeschmolzen, mit 80°C heißem Wasser vermischt und unter starkem Rühren emulgiert. Danach wurde die Emulsion unter schwachem Rühren abgekühlt. Bei einer Temperatur von 40°C wurden die restlichen Komponente unter Rühren zugegeben. Abschließend wurde mit AMP 95 ein pH-Wert von 7 eingestellt.

### Teilmischung B

| | |
|---|---|
| Ammoniumsulfat | 1,00g |
| Direktziehende Farbstoffe; | |
| gemäß Tabelle (I) (Beispiele D1-D5) | 1,00g-3,00g |
| Vitamin B6 (Pyridoxin HCl) | 1,00g |
| Wasser | 10,00g |

Der Farbstoff wurde in 50°C heißem Wasser unter Zugabe von Ammoniumsulfat, Weinsäure und Vitamin B6 (Pyridoxin·HCl) gelöst. Die Farbstofflösung (Teilmischung B) wurde zur Emulsion (Teilmischung A) gegeben und mit Wasser auf 100g aufgefüllt. Es wurde bis zum Erreichen der Raumtemperatur weitergerührt.

### FÄRBEMITTEL 2

### Teilmischung A

| | |
|---|---|
| Hydrenol^{®}D¹ | 1,50g |
| Kokoslorol^{®}C12-18² | 3,50g |
| Texapon^{®} NSO F³ | 15,00g |
| Polychol^{®}5⁴ | 0,40g |
| Luviskol^{®}K 30⁵ | 0,50g |
| Ammoniak (25%-ige wäßrige Lösung) | ad pH 9 |
| Wasser | 55,00g |

| | |
|---|---|
| ¹ C₁₆-C₁₈-Fettalkohol (INCI-Bezeichnung: Cetearyl alcohol) (COGNIS) ² C₁₂-C₁₈-Fettalkohol (INCI-Bezeichnung: Coconut alcohol) (COGNIS) ³ Laurylethersulfat Natrium Salz (INCI: Sodium Laureth Sulfate) (COGNIS) ⁴ Lanolinalkohol+5-EO (INCI-Bezeichnung: LANETH-5) (CRODA) ⁵ Polyvinylpyrrolidon (INCI: PVP) (BASF) | |

Die Substanzen Hydrenol^{®}D, Kokoslorol^{®}C12-18, Texapon^{®}NSO F und Polychol^{®}5 wurden bei 80°C aufgeschmolzen, mit 80°C heißem Wasser vermischt und unter starkem Rühren emulgiert. Danach wurde das AMPD und das Luviskol^{®}K 30 zugegeben und die Emulsion unter schwachem Rühren abgekühlt.

### Teilmischung B

| | |
|---|---|
| Ammoniumsulfat | 1,00g |
| Direktziehende Farbstoffe; gemäß Tabelle (I) (Beispiele D6-D10) | 1,00g-3,00g |
| Vitamin B6 (Pyridoxin·HCl) | 1,00g |
| Wasser | 10,00g |

Der Farbstoff wurde in 50°C heißem Wasser unter Zugabe von Ammoniumsulfat und gegebenenfalls etwas Ammoniak gelöst.
Die Farbstofflösung (Teilmischung B) wurde zur Emulsion (Teilmischung A) gegeben, mit Ammoniak auf pH=9 eingestellt und mit Wasser auf 100g aufgefüllt. Es wurde bis zum Erreichen der Raumtemperatur weitergerührt.

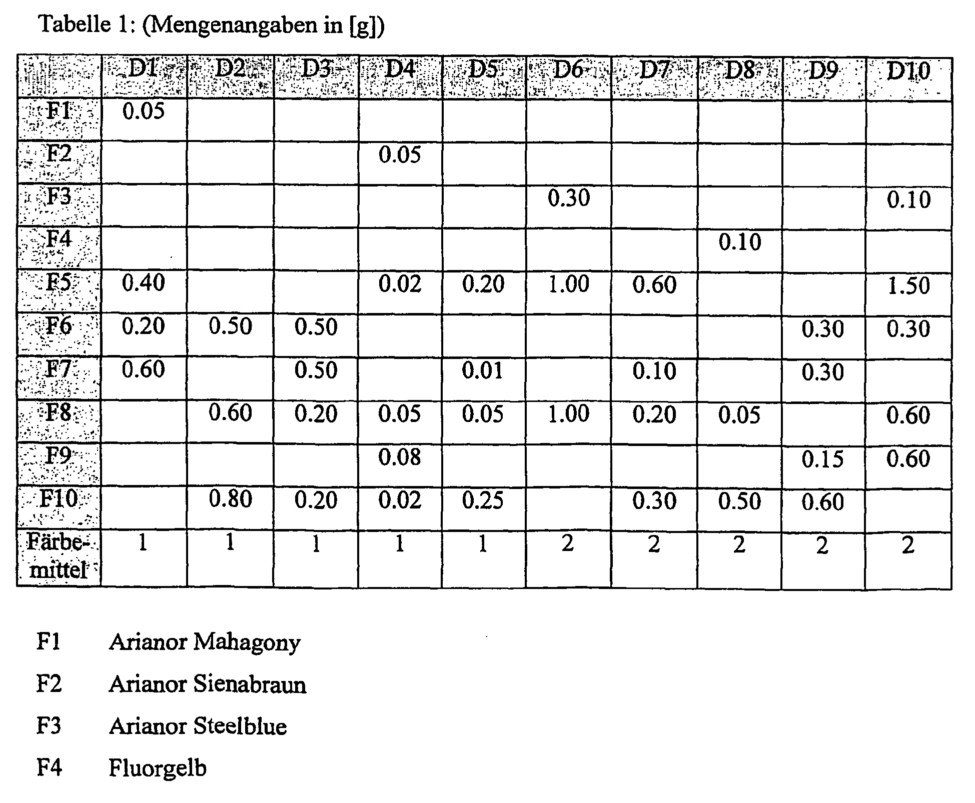

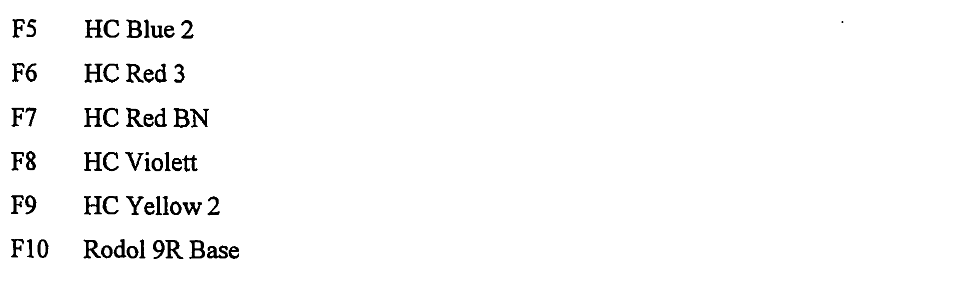

### AUSFÄRBUNGEN

Die so erhaltenen Färbemittel wurden je auf 5cm lange Strähnen standardisierten, zu 80% ergrauten, aber nicht besonders vorbehandelten Menschenhaares aufgetragen, und dort 30min bei 32°C belassen. Danach wurde das Haar gespült, mit einem üblichen Haarwaschmittel ausgewaschen und anschließend getrocknet.

Das Ergebnis der Färbeversuche ist der Tabelle 2 zu entnehmen.

**Tabelle 2**

| **Formulierung (siehe Tabelle 1)** | **Nuance des gefärbten Haares** |
|---|---|
| D1 | Granatbraun |
| D2 | Rubinrot |
| D3 | Violettrot |
| D4 | Weizen |
| D5 | Haselnuß |
| D6 | Dunkelviolett |
| D7 | Schoko |
| D8 | Lehmfarben |
| D9 | Kupfer |
| D10 | Braun |

### II) Nachweis der strukturgebenden Wirkung von Vitamin B6 bei gleichzeitiger Applikation von Vitamin B6 mit dem Färbemittel

### A) Verwendete Analysemethode: HP-DSC (High Pressure Differential Scanning Calorimetry)

Thermoanalytische Untersuchungen eignen sich besonders zur Charakterisierung von Zweiphasensystemen, zu denen die Humanhaare als Faserkeratine mit ihrem kristallinen α-Helix-Anteil und amorphen Matrix-Anteil ebenfalls gehören. Auf der einen Seite können Glasübergänge und Alterungsverhalten der amorphen Matrix untersucht werden, auf der anderen Seite liefert das Schmelzverhalten der kristallinen, helicalen Phase wichtige Erkenntnisse. Thermoanalytische Untersuchungen sind erstmals 1899 beschrieben, erste Differenzthermoanalysen (DTA) an Proteinfasern wurden Ende der fünfziger Jahre durchgeführt (F. Schwenker, J.H. Dusenbury, Text. Res. J. 1963, 30, Seite 800 ff; W. D. Felix, M.A. McDowall, H. Eyring, ibid. (1963), 33, Seite 465 ff.). In den folgenden Jahren sind unterschiedliche thermoanalytische Meßverfahren, wie DTA, HP-DTA (High Pressure, Hochdruck-DTA) und DSC (Differential Scanning Calorimetry, Dynamische Differenz-Kalorimetrie), an Keratinfasern angewendet worden um z.B. das Phänomen der Superkontraktion, α-β-Phasenübergänge der Helices oder Denaturierungsvorgänge zu untersuchen. In jüngster Zeit wird, insbesondere am Deutschen Wollforschungsinstitut in Aachen (F.J. Wortmann, H. Deutz, J. Appl. Polym. Sci. 1993, 48, Seite 137ff.), die Methode der IIP-DSC zur Untersuchung von Keratinfasem genutzt, die die Probleme mit pyrolytischen Effekten, wie sie bei der konventionellen DSC auftreten, und Probleme mit der Datenerfassung und -interpretation, wie sie die DTA birgt, ausschließt. Dabei werden DSC-Messungen an Keratinen durchgeführt, die mit Wasser in kommerziell erhältlichen, druckfesten Meßkapseln eingeschlossen sind. Im Keratin-Wasser-System entwickelt sich beim Erhitzen oberhalb von 100°C in den verkapselten Stahltiegeln ein Wasserdampfhochdruck, woraus sich die HP-DSC Analyse ableitet. Der gravierende Unterschied der HP-DSC-Thermogramme von Humanhaaren im Vergleich zu normalen DSC-Thermogrammen ist der, daß die endothermen Peaks, die den Umwandlungspunkt und Umwandlungsenthalpie wiedergeben, hier um ca. 90 °C zu niedrigeren Temperaturen verschoben sind. Das rührt daher, daß das Wasser nach Diffusion in die Haarfaser durch Schwächung und Spaltung von Wasserstoffbrücken- und Salzbindungen die Proteinstabilität vermindert und so die "Verleimungstemperatur" der Keratine herabsetzt. Werden durch das superkontrahierende Agens wie Wasser nur Wasserstoffbrücken und Salzbrücken gelöst, so ist der thermische Effekt reversibel (Superkontraktion). Der Vorgang wird jedoch irreversibel, sobald auch kovalente Bindungen, wie z. B. Disulfidbrücken, gespalten werden. Dies tritt ein, wenn man Humanhaarfasern in druckfesten Kapseln mit Wasser auf über 150 °C erhitzt. Die irreversible Umwandlung, interpretiert als Übergang der α-helicalen Bereiche in den Proteinen in einen ungeordneten Zustand, resultiert in endothermen Peaks, wobei die Peaklage den Umwandlungs- oder auch Denaturierungspunkt und die Peakfläche die Umwandlungs- oder Denaturierungs-Enthalpie wiedergibt.

Unter Verwendung der Dynamischen Differenz-Kalorimetrie (DSC) können demnach strukturelle sowie chemische Zustände und Veränderungen in Faserkeratinen und insbesondere in Humanhaaren erfaßt werden. Unter genau definierten Versuchsbedingungen kann man bei Humanhaaren die kalorimetrisch erfaßbaren Vorgänge anhand von Thermogrammen aufzeichnen und sie bezüglich der Peaklagen, - strukturen und -flächen als Indikator für die Beeinflussung von Ordnungs-Unordnungsübergangen durch Änderungen innerer und/oder äußerer Parameter, hervorrufen z. B. durch kosmetische Behandlung der Haare, verwenden. D. h. aus den im Thermogramm von Humanhaaren aufgezeichneten endothermen Peaks lassen sich aufgrund von Peaklage (Umwandlungspunkt) und Peakfläche (Umwandlungsenthalpie) Aussagen über Festigkeit bzw. Schädigung der Humanhaarfaser treffen.

Ausführliche Untersuchungen bezüglich des Einflusses des Cystingehaltes auf die Denaturierung der α-Helices in Keratinen haben z. B. gezeigt, daß die Denaturierungs-Temperatur (Übergangstemperatur) des Keratins linear mit dem Cystingehalt ansteigt. Die erhöhte Stabilität des Matrixbereichs aufgrund des höheren Vernetzungsgrades des erhöhten Anteils an Disulfidbrücken in der Matrix führt dazu, daß die Umwandlung der in diese Matrix eingebetteten Helices erschwert wird und resultiert somit in einer Erhöhung der Denaturierungs-Temperatur. Umgekehrt kann in der Regel eine Denaturierungs-Temperatur- und vor allem Denaturierungs-Enthalpieerniedrigung bei durch Dauerwelle oder Bleichung bzw. Färbung behandelten Humanhaaren beobachtet werden (H. Deutz, Doktorarbeit, RWTH Aachen 1993).

### B) Durchführung

Humanhaar (Alkinco 6633) wurde gezielt durch eine Dauerwellbehandlung (Marktprodukt Poly Lock extra starke Dauerwelle; 40 Minuten Dauerwelle, 10 Minuten Fixieren) geschädigt. Anschließend wurde das vorbehandelte Haar mittels eines Färbemittels mit unterschiedlichem Gehalt an Pyridoxin·HCl gefärbt. Die Denaturierungstemperaturen der eingefärbten Haarproben wurden per HP-DSC thermoanalytisch bestimmt.

4,0 g der nach I) präparierten Mittel D2 und D8 (jeweils mit 1% Pyridoxin und ohne Pyridoxin) wurden auf 0,5 g geschädigtes Humanhaar aufgetragen. Nach 30 Minuten Einwirkungszeit bei 32°C wurde das Haar gespült und anschließend getrocknet. Mittels HP-DSC-Messungen wurde dann auf restrukturierende Effekte der Wirkstoffe geprüft. Die erhaltenen Denaturierungstemperaturen sind in Tabelle 3 aufgelistet.

**Tabelle 3:**

| **Beispiel** | **Formulierung** | **Denaturierungstemperatur [°C]** |
|---|---|---|
| II.1 | keine (unbehandeltes Haar) | 145.8 |
| II.2 | D2 ohne Pyridoxin·HCl | 146.0 |
| II.3 | D2 | 148.3 |
| II.4 | D8 ohne Pyridoxin·HCl | 146.2 |
| II.5 | D8 | 148.7 |

## Patentansprüche

1. Mittel zum Färben von Keratinfasern, insbesondere dem menschlichen Haar enthaltend mindestens einen direktziehenden Farbstoff, **dadurch gekennzeichnet, daß** das Färbemittel zusätzlich mindestens eine Verbindung der Formel (I) 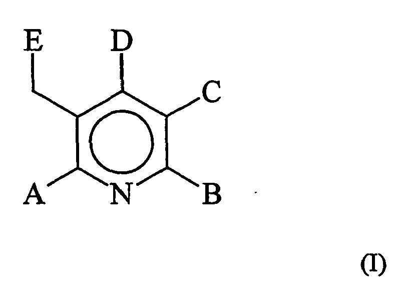 worin
- A und B stehen unabhängig voneinander für Wasserstoff, Halogen, eine C₁-C₄-Alkylgruppe, eine C₃-C₆-Cycloalkylgruppe, eine C₁-C₄-Monohydroxyalkylgruppe, eine C₂-C₄-Oligohydroxyalkylgruppe, eine C₁-C₄-Aminoalkylgruppe, eine Gruppe -OR oder eine Gruppe -NR¹R², in der R¹ und R² unabhängig voneinander für Wasserstoff, eine C₁-C₄-Alkylgruppe oder eine C₁-C₄-Monohydroxyalkylgruppe stehen, oder R¹ und R² zusammen mit dem Stickstoffatom einen gesättigten Ring bilden,
- C steht für eine Gruppe -OR, -NR¹R², -OP(O)(OR³)₂, eine C₁-C₄-Monohydroxyalkylgruppe, eine C₂-C₄-Oligohydroxyalkylgruppe oder eine C₁-C₄-Alkylgruppe,
- D steht für eine Gruppe -OR, eine Carboxygruppe, eine C₁-C₂₂-Alkoxycarbonylgruppe, einen Formylrest, eine Gruppe -CH₂OR oder eine Gruppe - CH₂-NR₂,
- E steht für eine Gruppe -OR, -OP(O)(OR³)₂, eine C₁-C₄-Monohydroxyalkylgruppe oder eine C₂-C₄-Oligohydroxyalkylgruppe,
wobei
- R jeweils steht für Wasserstoff, eine C₁-C₄-Alkylgruppe, eine C₁-C₂₂-Acylgruppe, eine Hydroxy-C₂-C₂₂-acylgruppe, eine C₂-C₁₀-Carboxyacylgruppe, eine C₃-C₁₀-Oligocarboxyacylgruppe, eine Oligocarboxymonohydroxy-C₃-C₁₀-acylgruppe, eine Oligocarboxy-oligohydroxy-C₃-C₁₀-acylgruppe, eine C₃-C₈-Cycloalkylgruppe, eine C₁-C₄-Monohydroxyalkylgruppe, eine C₂-C₄-Oligohydroxyalkylgruppe, eine Arylgruppe, welche eine Hydroxy-, Nitro- oder Aminogruppe enthalten kann, einen heteroaromatischen Rest oder eine Gruppe -CH₂CH₂NR¹R², in der R¹ und R² wie oben definiert sind,
- R³ jeweils steht für Wasserstoff oder eine C₁-C₅-Alkylgruppe,
oder eines der entsprechenden physiologisch verträglichen Salze enthält.

2. Mittel nach Anspruch 1, **dadurch gekennzeichnet, daß** eine der beiden Gruppen A oder B für Wasserstoff steht.

3. Mittel nach einem der Ansprüche 1 oder 2, **dadurch gekennzeichnet, daß** eine der beiden Gruppen A oder B Wasserstoff und die andere Gruppe eine C₁-C₄-Alkylgruppe ist.

4. Mittel nach einem der Ansprüche 1 bis 3, **dadurch gekennzeichnet, daß** C für eine Hydroxygruppe, eine C₁-C₄-Monohydroxyalkylgruppe oder eine C₂-C₄-Oligohydroxyalkylgruppe steht.

5. Mittel nach einem der Ansprüche 1 bis 4, **dadurch gekennzeichnet, daß** D für eine Hydroxymethylgruppe, eine Hydroxygruppe, eine Carboxygruppe, eine Gruppe - CH₂-NR₂ oder einen Formylrest steht.

6. Mittel nach einem der Ansprüche 1 bis 5, **dadurch gekennzeichnet, daß** E für eine Hydroxygruppe, eine C₁-C₄-Monohydroxyalkylgruppe oder eine Gruppe - OP(O)(OH)₂ steht.

7. Mittel gemäß einer der Ansprüche 1 bis 6, **dadurch gekennzeichnet, daß** die Verbindung gemäß Formel (I) aus der Gruppe Pyridoxin, Pyridoxal, Pyridoxal-5'-phosphat und Pyridoxamin ausgewählt ist.

8. Mittel nach einem der Ansprüche 1 bis 7, **dadurch gekennzeichnet, daß** der direktziehende Farbstoff ausgewählt ist aus HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 12, Disperse Blue 3, HC Violet 1, Disperse Violet 1, Disperse Violet 4, Acid Violet 43, Disperse Black 9, Acid Black 52, 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

9. Mittel nach einem der Ansprüche 1 bis 8, **dadurch gekennzeichnet, daß** es zusätzlich mindestens ein Oxidationsfarbstoffvorprodukt vom Entwicklertyp und/gegebenenfalls mindestens ein Oxidationsfarbstoffvorprodukt vom Kupplertyp enthält.

10. Verfahren zum Tönen und/oder Färben von Keratinfasern, insbesondere menschlichen Haaren, in dem
- gewünschtenfalls ein Vorbehandlungsmittel M1 auf die Faser aufgebracht wird, dann
- ein Tönungs- oder Färbemittel M2 auf der Faser zur Anwendung kommt, wobei gewünschtenfalls dem Mittel M2 vor der Anwendung ein weiteres Mittel M3 zugegeben wird.
- dieses Färbemittel M2 nach einer Zeit von 5-30 Minuten von der Faser abgespült wird,
- und nach der Behandlung gegebenenfalls ein Nachbehandlungsmittel M4 auf die Faser aufgetragen und nach einer Einwirkzeit von einigen Minuten wieder abgespült wird,
**dadurch gekennzeichnet, daß** mindestens eines der Mittel M1, M2, M3 oder M4 mindestens eine Verbindung gemäß Formel (I) enthält.

11. Verfahren nach Anspruch 10, **dadurch gekennzeichnet, daß** dem Mittel M2 kurz vor der Anwendung ein Mittel M3, enthaltend mindestens eine Verbindung gemäß Formel (I), zugegeben wird.

12. Verfahren nach einem der Ansprüche 10 oder 11, **dadurch gekennzeichnet, daß** das Mittel M2 eine Zubereitung gemäß einem der Ansprüche 1 bis 9 ist.

13. Verwendung eines Mittels gemäß Anspruch 1 in einem Verfahren zum Tönen und/oder Färben von Keratinfasern, insbesondere dem menschlichen Haar.

## Claims

1. A preparation for coloring keratin fibers, more particularly human hair, containing at least one substantive dye, **characterized in that** the colorant additionally contains at least one compound corresponding to formula (I): 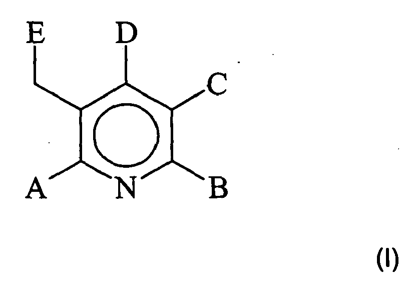 in which
- A and B independently of one another represent hydrogen, halogen, a C₁₋₄ alkyl group, a C₃₋₆ cycloalkyl group, a C₁₋₄ monohydroxyalkyl group, a C₂₋₄ oligohydroxyalkyl group, a C₁₋₄ aminoalkyl group, a group -OR or a group -NR¹R², where R¹ and R² independently of one another represent hydrogen, a C₁₋₄ alkyl group or a C₁₋₄ monohydroxyalkyl group or R¹ and R² together with the nitrogen atom form a saturated ring,
- C represents a group -OR, -NR¹R², -OP(O)(OR³)₂, a C₁₋₄ monohydroxyalkyl group, a C₂₋₄ oligohydroxyalkyl group or a C₁₋₄ alkyl group,
- D represents a group -OR, a carboxy group, a C₁₋₂₂ alkoxycarbonyl group, a formyl group, a group -CH₂OR or a group -CH₂-NR₂,
- E represents a group -OR, -OP(O)(OR³)₂, a C₁₋₄ monohydroxyalkyl group or a C₂₋₄ oligohydroxyalkyl group,
- R representing hydrogen, a C₁₋₄ alkyl group, a C₁₋₂₂ acyl group, a hydroxy-C₂₋₂₂-acyl group, a C₂₋₁₀ carboxyacyl group, a C₃₋₁₀ oligocarboxyacyl group, an oligocarboxymonohydroxy-C₃₋₁₀-acyl group, an oligocarboxyoligohydroxy-C₃₋₁₀-acyl group, a C₃₋₈ cycloalkyl group, a C₁₋₄ monohydroxyalkyl group, a C₂₋₄ oligohydroxyalkyl group, an aryl group which may contain a hydroxy, nitro or amino group, a heteroaromatic group or a group -CH₂CH₂NR¹R², where R¹ and R² are as defined above,
- R³ representing hydrogen or a C₁₋₅ alkyl group,
or one of the corresponding physiologically compatible salts.

2. A colorant claimed in claim 1, **characterized in that** one of the two groups A and B is hydrogen.

3. A colorant claimed in claim 1 or 2, **characterized in that** one of the two groups A and B is hydrogen and the other is a C₁₋₄ alkyl group.

4. A colorant claimed in any of claims 1 to 3, **characterized in that** C is a hydroxy group, a C₁₋₄ monohydroxyalkyl group or a C₂₋₄ oligohydroxyalkyl group.

5. A colorant claimed in any of claims 1 to 4, **characterized in that** D is a hydroxymethyl group, a hydroxy group, a carboxy group, a group -CH₂-NR₂ or a formyl group.

6. A colorant claimed in any of claims 1 to 5, **characterized in that** E is a hydroxy group, a C₁₋₄ monohydroxyalkyl group or a group -OP(O)(OH)₂.

7. A colorant claimed in any of claims 1 to 6, **characterized in that** the compound corresponding to formula (I) is selected from the group consisting of pyridoxine, pyridoxal, pyridoxal-5'-phosphate and pyridoxamine.

8. A colorant as claimed in any of claims 1 to 7, **characterized in that** the substantive dye is selected from HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 12, Disperse Blue 3, HC Violet 1, Disperse Violet 1, Disperse Violet 4, Acid Violet 43, Disperse Black 9 and Acid Black 52 and also 1,4-diamino-2-nitrobenzene, 2-amino-4-nitrophenol, 1,4-bis-(β-hydroxyethyl)-amino-2-nitrobenzene, 3-nitro-4-(β-hydroxyethyl)-aminophenol, 2-(2'-hydroxyethyl)-amino-4,6-dinitrophenol, 1-(2'-hydroxyethyl)-amino-4-methyl-2-nitrobenzene, 1-amino-4-(2'-hydroxyethyl)-amino-5-chloro-2-nitrobenzene, 4-amino-3-nitrophenol, 1-(2'-ureidoethyl)-amino-4-nitrobenzene, 4-amino-2-nitrodiphenylamine-2'-carboxylic acid, 6-nitro-1,2,3,4-tetrahydroquinoxaline, 2-hydroxy-1,4-naphthoquinone, picramic acid and salts thereof, 2-amino-6-chloro-4-nitrophenol, 4-ethylamino-3-nitrobenzoic acid and 2-chloro-6-ethylamino-1-hydroxy-4-nitrobenzene.

9. A composition as claimed in any of claims 1 to 8, **characterized in that** it additionally contains at least one oxidation dye precursor of the primary intermediate type and optionally at least one oxidation dye precursor of the secondary intermediate type.

10. A process for tinting and/or coloring keratin fibers, more particularly human hair, in which
- if desired, a pretreatment preparation M1 is applied to the fibers,
- a tint or colorant M2 is then used on the fibers, another preparation M3 optionally being added to M2 before application,
- the colorant M2 is rinsed from the fibers after a contact time of 5 to 30 minutes and
- after the treatment, an aftertreatment preparation M4 is optionally applied to the fibers and is rinsed from them after a contact time of a few minutes,
**characterized in that** at least one of the preparations M1, M2, M3 or M4 contains at least one compound corresponding to formula (I).

11. A process as claimed in claim 1, **characterized in that** a preparation M3 containing at least one compound corresponding to formula (I) is added to the colorant M2 shortly before application.

12. A process as claimed in claim 11 or 12, **characterized in that** the colorant M2 is a preparation according to any of claims 1 to 9.

13. The use of the preparation claimed in claim 1 in a process for tinting and/or coloring keratin fibers, more particularly human hair.

## Revendications

1. Agent pour la teinture de fibres kératiniques, en particulier de cheveux humains, contenant au moins un colorant montant directement sur la fibre, **caractérisé en ce que** l'agent de teinture contient au moins un composé de formule (I) 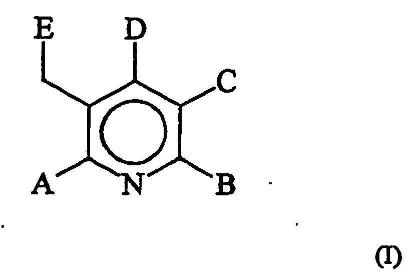 dans laquelle
- A et B représentent, indépendamment l'un de l'autre, hydrogène, halogène, un groupe C₁-C₄-alkyle, un groupe C₃-C₆-cycloalkyle, un groupe C₁-C₄-monohydroxyalkyle, un groupe C₂-C₄-oligohydroxyalkyle, un groupe C₁-C₄-aminoalkyle, un groupe -OR ou un groupe -NR¹R², où R¹ et R² représentent, indépendamment l'un de l'autre, hydrogène, un groupe C₁-C₄-alkyle ou un groupe C₁-C₄-monohydroxyalkyle, ou R¹ et R² forment ensemble avec l'atome d'azote un cycle saturé,
- C représente un groupe -OR, -NR¹R², -OP(O)(OR³)₂, un groupe C₁-C₄-monohydroxyalkyle, un groupe C₂-C₄-oligohydroxyalkyle ou un groupe C₁-C₄-alkyle,
- D représente un groupe -OR, un groupe carboxy, un groupe C₁-C₂₂-alcoxycarbonyle, un radical formyle, un groupe -CH₂OR ou un groupe -CH₂-NR₂,
- E représente un groupe -OR, -OP(O)(OR³)₂, un groupe C₁-C₄-monohydroxyalkyle ou un groupe C₂-C₄-oligohydroxyalkyle,
où :
- R représente à chaque fois hydrogène, un groupe C₁-C₄-alkyle, un groupe C₁-C₂₂-acyle, un groupe hydroxy-C₂-C₂₂-acyle, un groupe C₂-C₁₀-carboxyacyle, un groupe C₃-C₁₀-oligocarboxyacyle, un groupe oligocarboxy-monohydroxy-C₃-C₁₀-acyle, un groupe oligocarboxy-oligohydroxy-C₃-C₁₀-acyle, un groupe C₃-C₈-cycloalkyle, un groupe C₁-C₄-monohydroxyalkyle, un groupe C₂-C₄-oligohydroxyalkyle, un groupe aryle, qui peut contenir un groupe hydroxy, nitro ou amino, un radical hétéroaromatique ou un groupe -CH₂CH₂NR¹R², dans lequel R¹ et R² sont tels que définis ci-dessus,
- R³ représente à chaque fois hydrogène ou un groupe C₁-C₅-alkyle,
ou un des sels physiologiquement acceptables correspondants.

2. Agent selon la revendication 1, **caractérisé en ce qu'**un des deux groupes A ou B représente hydrogène.

3. Agent selon l'une quelconque des revendications 1 ou 2, **caractérisé en ce qu'**un des deux groupes A ou B représente hydrogène et l'autre groupe est un groupe C₁-C₄-alkyle.

4. Agent selon l'une quelconque des revendications 1 à 3, **caractérisé en ce que** C représente un groupe hydroxy, un groupe C₁-C₄-monohydroxyalkyle ou un groupe C₂-C₄-oligohydroxyalkyle.

5. Agent selon l'une quelconque des revendications 1 à 4, **caractérisé en ce que** D représente un groupe hydroxyméthyle, un groupe hydroxy, un groupe carboxy, un groupe -CH₂-NR₂ ou un radical formyle.

6. Agent selon l'une quelconque des revendications 1 à 5, **caractérisé en ce que** E représente un groupe hydroxy, un groupe C₁-C₄-monohydroxyalkyle ou un groupe -OP(O)(OH)₂.

7. Agent selon l'une quelconque des revendications 1 à 6, **caractérisé en ce que** le composé selon la formule (I) est choisi dans le groupe formé par la pyridoxine, le pyridoxal, le pyridoxal-5'-phosphate et la pyridoxamine.

8. Agent selon l'une quelconque des revendications 1 à 7, **caractérisé en ce que** le colorant montant directement sur la fibre est choisi parmi le HC Yellow 2, le HC Yellow 4, le HC Yellow 5, le HC Yellow 6, le HC Yellow 12, le HC Orange 1, le Disperse Orange 3, le HC Red 1, le HC Red 3, le HC Red 10, le HC Red 11, le HC Red 13, le HC Red BN, le HC Blue 2, le HC Blue 12, le Disperse Blue 3, le HC Violet 1, le Disperse Violet 1, le Disperse Violet 4, l'Acid Violet 43, le Disperse Black 9, l'Acid Black 52, le 1,4-diamino-2-nitrobenzène, le 2-amino-4-nitrophénol, le 1,4-bis-(β-hydroxyéthyl)-amino-2-nitrobenzène, le 3-nitro-4-(β-hydroxyéthyl)-aminophénol, le 2-(2'-hydroxyéthyl)amino-4,6-dinitrophénol, le 1-(2'-hydroxyéthyl)amino-4-méthyl-2-nitrobenzène, le 1-amino-4-(2'-hydroxyéthyl)-amino-5-chloro-2-nitrobenzène, le 4-amino-3-nitrophénol, le 1-(2'-uréidoéthyl)amino-4-nitrobenzène, l'acide 4-amino-2-nitrodiphénylamine-2'-carboxylique, la 6-nitro-1,2,3,4-tétrahydroquinoxaline, la 2-hydroxy-1,4-naphtoquinone, l'acide picramique et ses sels, le 2-amino-6-chloro-4-nitrophénol, l'acide 4-éthylamino-3-nitrobenzoïque et le 2-chloro-6-éthylamino-1-hydroxy-4-nitrobenzène.

9. Agent selon l'une quelconque des revendications 1 à 8, **caractérisé en ce qu'**il contient en outre au moins un précurseur d'un colorant d'oxydation du type agent de développement et/le cas échéant au moins un précurseur de colorant d'oxydation du type agent de couplage.

10. Procédé pour la coloration et/ou la teinture de fibres kératiniques, en particulier de cheveux humains, dans lequel
- on applique, si souhaité, un agent de prétraitement M1 sur les fibres, puis
- on utilise un agent de coloration ou de teinture M2 sur les fibres, l'agent M2 étant si souhaité mélangé avant l'utilisation avec un autre agent M3.
- cet agent de teinture M2 est éliminé des fibres par rinçage après un laps de temps de 5-30 minutes,
- et on applique après le traitement le cas échéant un agent de post-traitement M4 sur les fibres qui est à nouveau éliminé par rinçage après un temps d'action de quelques minutes,
**caractérisé en ce qu'**au moins un des agents M1, M2, M3 ou M4 contient au moins un composé selon la formule (I).

11. Procédé selon la revendication 10, **caractérisé en ce que** l'agent M2 est mélangé peu avant l'utilisation avec un agent M3 contenant au moins un composé selon la formule (I).

12. Procédé selon l'une quelconque des revendications 10 ou 11, **caractérisé en ce que** l'agent M2 est une préparation selon l'une quelconque des revendications 1 à 9.

13. Utilisation d'un agent selon la revendication 1 dans un procédé pour la coloration et/ou la teinture de fibres kératiniques, en particulier les cheveux humains.
